(19)

Europäisches Patentamt
European Patent Office
Office européen des brevets

(11) **EP 4 112 169 A1**

(12) **EUROPEAN PATENT APPLICATION**

(43) Date of publication:
**04.01.2023 Bulletin 2023/01**

(21) Application number: **21183582.2**

(22) Date of filing: **03.07.2021**

(51) International Patent Classification (IPC):
**B01J 35/00** (2006.01)       **B01J 23/89** (2006.01)
**B01J 31/24** (2006.01)       **B01J 31/20** (2006.01)
**C07C 29/156** (2006.01)       **C07C 45/50** (2006.01)

(52) Cooperative Patent Classification (CPC):
(C-Sets available)
**B01J 35/0006; B01J 23/8913; B01J 23/894;**
**B01J 23/8946; B01J 31/20; B01J 31/2404;**
**C07C 29/156;** B01J 21/04; B01J 2231/321;
B01J 2231/641; B01J 2531/845        (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Studiengesellschaft Kohle mbH**
  **45470 Mülheim (DE)**
• **Max-Planck-Gesellschaft zur Förderung der**
  **Wissenschaften e.V.**
  **80539 München (DE)**

(72) Inventors:
• **LEITNER, Walter**
  **52074 Aachen (DE)**

• **JESKE, Kai**
  **44803 Bochum (DE)**
• **PRIETO, Gonzalo**
  **46015 Valencia (ES)**
• **VORHOLT, Andreas Johannes**
  **45239 Essen (DE)**
• **RÖSLER, Thorsten**
  **45473 Mülheim an der Ruhr (DE)**
• **BELLEFLAMME, Maurice**
  **45468 Mülheim an der Ruhr (DE)**

(74) Representative: **Nobbe, Matthias**
**Demski & Nobbe**
**Patentanwälte**
**Mülheimer Strasse 210**
**47057 Duisburg (DE)**

(54) **PROCESS FOR CONVERTING SYNTHESIS GAS TO HIGHER ALCOHOLS**

(57)      The present invention refers to a process for converting a feed gas stream comprising carbon monoxide and hydrogen as major components (synthesis gas) into higher ($C_{3+}$) alcohols making use of a catalyst combination of a Fischer-Tropsch catalyst and an olefin hydroformylation catalyst. In a second aspect, the invention relates to a Fischer-Tropsch catalyst suitable to be applied in said process.

Figure 1

EP 4 112 169 A1

(52) Cooperative Patent Classification (CPC): (Cont.)

C-Sets
**C07C 29/156, C07C 31/02;**
**C07C 29/156, C07C 31/10**

**Description**

The present invention refers, in a first aspect, to a process for converting a feed gas stream comprising carbon monoxide and hydrogen as major components (synthesis gas) into higher ($C_{3+}$) alcohols making use of a catalyst combination of a Fischer-Tropsch catalyst and an olefin hydroformylation catalyst. In a second aspect, the invention relates to a Fischer-Tropsch catalyst suitable to be applied in said process.

Higher alcohols, also known as oxo-alcohols, that is, alcohol compounds with an unsaturated hydrocarbon molecular backbone containing three or more carbon atoms and a single alcohol functionality, are highly demanded chemicals. When the hydrocarbon molecular backbone is unbranched and the alcohol functionality is located at a terminal position, the compounds are denoted as terminal higher alcohols. Higher alcohols with a hydrocarbon chain containing 4-7 carbon atoms in their molecular structure find application as solvents or precursors thereof. Higher alcohols with a hydrocarbon chain containing 8-12 carbon atoms are valuable as precursors for plasticizers. Higher alcohols with yet longer hydrocarbon chains containing 12 or more carbon atoms are valuable as raw materials for the manufacture of detergents and auxiliary compounds in the textile industry. Higher oxo-alcohols are typically manufactured by the hydroformylation reaction (or oxo-synthesis) of olefins with carbon monoxide (CO) into aldehyde compounds, followed by subsequent hydrogenation with hydrogen. If both the reaction of hydroformylation of an olefin to the corresponding aldehyde and the reaction of hydrogenation of said aldehyde to an alcohol final product take place in a single process step, the transformation is typically referred to as reductive hydroformylation. A good survey for the hydroformylation of olefins for the manufacture of higher alcohols is Hydroformylation: Fundamentals, Processes, and Applications in Organic Synthesis, Eds. Arminand Börner Robert Franke, 2016, ISBN:9783527335527.

Synthesis gas (often abbreviated as "syngas") is a mixture typically composed of carbon monoxide (CO), hydrogen ($H_2$) and, in certain cases, also carbon dioxide ($CO_2$) as major components. Synthesis gas streams might additionally comprise other gases such as nitrogen ($N_2$), helium (He), argon (Ar), water steam ($H_2O$), or light hydrocarbons such as methane ($CH_4$), ethane ($C_2H_6$), propane ($C_3H_8$), which do not alter the reactivity of the major components to a significant extent. Syngas is an important and versatile commodity which can be obtained from a large variety of carbonaceous feedstocks, e.g. via steam reforming or partial oxidation of natural gas, via gasification of coal, via gasification and/or reforming of biomass feedstocks, via hydrogenation of carbon dioxide, among others. Syngas might be converted on the surface of a solid catalyst via a surface polymerization reaction, the so-called Fischer-Tropsch synthesis, into a mixture of mostly *normal (unbranched)* hydrocarbons. This reaction takes place on catalysts containing cobalt (Co), ruthenium (Ru), or iron (Fe) as the major catalytically active species, or precursors thereof, either in an unsupported (bulk) fashion or dispersed in the form of nanoparticles on a porous carrier, and possibly incorporating further components which play the role of structural and catalytic promoters to improve catalyst activity, selectivity or stability under standard operation conditions.

The reaction proceeds via a surface chain-growth polymerization mechanism and thus results in primarily linear hydrocarbon products, which typically obey a so-called Anderson-Schulz-Flory (ASF) chain-length distribution. According to this distribution, the hydrocarbon products span a wide range of hydrocarbon chain-lengths, typically from $C_1$ to $C_{50+}$, where the subscripts indicate the number of carbon atoms in the hydrocarbon molecule, and obey the following mathematical relationship:

$$W_n = n \cdot (1 - \alpha)^2 \cdot \alpha^{n-1}$$

where $W_n$ is the mass fraction of reaction products with *n* carbon atoms in their hydrocarbon chain formula and $\alpha$ (alpha) stands for the chain growth probability factor. In an ASF distribution, alpha serves to describe the production distribution and it represents the probability, on a 0 to 1 basis, of a hydrocarbon chain to undergo addition of a monomeric species, and thus grow in a carbon atom, under the reaction conditions. Experimentally, the chain growth probability (alpha) can be determined according to the relation $\alpha = e^m$, where *e* is Euler's number and *m* represents the slope obtained for the linear regression mathematical function of experimental reaction product quantification data in a plot representing $Ln(W_n/n)$ versus *n* in a sufficiently broad range of carbon chain lengths *n*, wherein *Ln* represents the natural logarithm function.

It is consensually believed that terminal alkenes (1-olefins, also known as alpha-olefins) are a major primary product of the Fischer-Tropsch reaction. However, these primary products are rather reactive under the reaction conditions, which are industrially relevant for this reaction, and typically undergo secondary reactions already before they egress from the Fischer-Tropsch catalyst particle where they formed. Such secondary processing might involve the re-adsorption of the alpha-olefin primary products on surface sites of the Fischer-Tropsch catalyst followed by secondary reactions such as double-bond isomerization into internal olefins, incorporation into growing hydrocarbon chains and/or their hydrogenation into the corresponding *n*-paraffins (alkanes). Since *n*-paraffins are significantly less reactive under the standard operation conditions, once they form, they typically do not undergo further transformations on a Fischer-Tropsch catalyst.

Two major families of Fischer-Tropsch catalysts are known in the state of the art. In a first family of catalysts, cobalt, ruthenium or a combination thereof are the major active metals. These catalysts are capable to operate at relatively mild temperatures, in the range of 423-523 K and are particularly active for hydrogenation reactions. Hence, the hydrocarbon product consists essentially of linear saturated $n$-paraffins, with only limited contributions from $C_{2+}$ olefins and oxygenate compounds. A second family of Fischer-Tropsch catalysts comprise iron carbide as the major active compound. These catalysts require comparably higher operation temperatures, typically >513 K. In this case, hydrogenation activity can be adjusted to obtain hydrocarbon products with significant contributions from linear $C_{2+}$ olefins. However, these catalysts are also active for the water-gas-shift reaction (WGSR), a reversible reaction, which converts equimolar amounts of carbon monoxide and water (generated as product of the Fischer-Tropsch reaction) into carbon dioxide and hydrogen according to the following reaction equation: $CO+H_2O \rightleftarrows CO_2+H_2$. Therefore, the selectivity of the Fischer-Tropsch conversion process to carbon dioxide is typically higher than 10%, and often higher than 30%.

The conventional route from syngas to higher alcohols involves a multi-stage process such as those described in documents EP1658254A2, US7674940B2, WO2002100806A1 and CN107744810A. In said processes, a syngas feed is contacted, in a first process step, with a Fischer-Tropsch catalyst, in a first reactor. Said Fischer-Tropsch catalyst is selected to be based on iron carbide as the major active component, and it is operated at a temperature in the range of 513-593 K and under a syngas pressure of 5-150 bar. Under these conditions, a mixture of hydrocarbon products comprising $C_{2+}$ olefins can be obtained as product from the Fischer-Tropsch reaction. Subsequently, the outlet stream from the Fischer-Tropsch process step, which comprises a mixture of hydrocarbon products, unreacted syngas, water and carbon dioxide is cooled down, the liquid hydrocarbon products condensed and alternatively fractionated via distillation operations. In a subsequent process step, the liquid hydrocarbon products from the Fischer-Tropsch process is contacted with a reductive hydroformylation catalyst and additional syngas in a second reactor to selectively convert the share of olefins in said hydrocarbon mixture into higher alcohols having one extra carbon atom in their molecular structure compared to the corresponding olefin educts. Said reductive hydroformylation catalyst is often selected to be a soluble coordination complex comprising a metal component -typically rhodium, cobalt or ruthenium- and an organic ligand, and it is operated at a temperature in the range of 323-453 K and under a syngas pressure of 50-200 bar. The operation temperature is limited to this range in order to prevent thermal decomposition and thus deactivation of the organometallic hydroformylation catalyst.

It is advantageous to develop a catalytic process for the selective production of higher alcohols from syngas in a single reactive step, avoiding intermediate separation, purification and conditioning operations. The document H.T. Luk et al. Status and prospects in higher alcohols synthesis from syngas, Chem. Soc. Rev., 2017,46, 1358-1426 serves as an exhaustive survey of catalysts and processes disclosed to convert syngas to higher alcohols in a single reactive step. Processes based on catalysts with copper as the major active component and additionally incorporating alkaline and alkaline earth elements -also known as alkalinized or modified methanol synthesis catalysts- as those disclosed in US4513100A, WO2012062338A1 and US4598061A promote the production of mainly branched and relatively short ($C_{6-}$) alcohols from syngas.

Processes based on catalysts with rhodium as the major active component, and possibly additional metal or lanthanide oxides, on a porous carrier, as those disclosed in US4096164A, CN1179993A and WO2006123150A1 result in high selectivities to $C_2$ alcohols, i.e. methanol and ethanol, but are not sufficiently efficient for the synthesis of higher alcohols.

Processes based on catalysts with molybdenum sulphide as the major active component, as those disclosed in WO2009032982A2, CN101535217B and WO2012078277A1 lead to selectivities to $C_{2+}$ alcohols lower than 50% and require the constant supply of a sulphur source in the syngas feed in order to sustain syngas conversion.

The production of higher alcohols as minority products in the Fischer-Tropsch synthesis is known from earlier developments of this reaction. State of the art documents disclose processes based on catalysts incorporating Fischer-Tropsch active metals such as cobalt and iron as the major active component for the conversion of syngas into higher alcohols. Under optimized process conditions, most of these processes are characterized by the production of higher alcohols, along with non-oxygenated hydrocarbons, which are in line with the ASF product distribution typical of the Fischer-Tropsch synthesis.

US4122110, US4291126 and Courty et al. "C1-C6 alcohols from synthesis gas on copper-cobalt catalysts" in J. Mol. Catal., 17 (1982) 241-254, disclose processes in which a syngas mixture is reacted on a catalyst comprising copper and cobalt as major active metals, and additional at least one metal oxide such as aluminium oxide, zinc oxide, chromium oxide, vanadium oxide or manganese oxide and at least one alkali or alkaline earth metal, to yield terminal higher alcohols.

Courty et al. "C1-C6 alcohols from synthesis gas on copper-cobalt catalysts" in J. Mol. Catal., 17 (1982) 241-254, discloses a process applying a catalyst containing copper, cobalt, aluminium oxide and sodium to be particularly selective to higher alcohols reaching a 29-31% selectivity (molar carbon basis) to $C_{2+}$ alcohols at a CO conversion level of 21-24%. However, alcohol chain length remained limited, with a chain growth probability (alpha) of 0.45.

Xiang et al. "Long-Chain Terminal Alcohols through Catalytic CO Hydrogenation" in J. Am. Chem. Soc.135 (2013) 7114-7117 describes a process in which catalysts based on copper and cobalt, as major active metals, and additionally manganese oxide are applied to convert a syngas ($H_2$/CO=2) to alcohols and hydrocarbons. Selectivities to total alcohols (including methanol) of 33.6-86 wt% could be obtained, with alcohol chain-growth probabilities 0.53-0.87, however, at limited CO conversion levels of 1-18%.

Xiang et al. "Higher alcohols through CO hydrogenation over CoCu catalysts: influence of precursor activation" in ACS Catal., 4, 8 (2014) 2792-2800, discloses a process in which catalysts based on copper and cobalt, as major active metals, are synthesized using an oxalate decomposition route and applied to convert a syngas ($H_2$/CO=1.5) at 40 bar and 513 K to alcohols and hydrocarbons. Selectivities to $C_{2+}$ alcohols up to 25.9 wt% were achieved, however at CO conversion levels equal or lower than 11% and with alcohol chain-growth probabilities equal or lower than 0.37.

Prieto et al. "Design and synthesis of copper-cobalt catalysts for the selective conversion of synthesis gas to ethanol and higher alcohols" in Angew. Chem. Int. Ed., 53 (2014) 6397-6401, describes the synthesis of catalysts containing copper and cobalt as major active metals, supported on molybdenum oxide and additionally alkalinized with potassium and their application to convert syngas ($H_2$/CO=2) into higher alcohols. An optimal catalyst composition, with an atomic ratio of copper to cobalt of 0.43 showed a selectivity of 23% (carbon molar basis) to $C_{2+}$ alcohols at CO conversion levels <5%. Alkalized catalysts based on copper and cobalt as described in this publication are active for the water-gas-shift-reaction, leading to high selectivities (22.4-41.6%) to the unwanted side-product carbon dioxide.

Zhao et al. "Insight into the Formation of Co@Co2C Catalysts for Direct Synthesis of Higher Alcohols and Olefins from Syngas" in ACS Catal. 8 (2018) 228-241 reports on catalysts based on cobalt and manganese supported on activated carbon for the conversion of syngas ($H_2$/CO=2) to alcohols and hydrocarbons. Selectivities to total alcohols of 17.5-21.4 % (carbon molar basis) are achieved at CO conversions of 10.5-40.5%, resulting in time-yields to total alcohols lower than 0.050 $g/g_{catalyst}$·h.

The inventors here started from the consideration that general limitations are associated to processes for the direct conversion of syngas to higher alcohols. The inventors have found that a first limitation relates to the fact that the selectivity of the process to higher alcohols decreases significantly with increasing the conversion degree of the syngas feed achieved in the reactor, limiting the overall time-yield to higher alcohols.

The inventors have additionally considered that a further technical limitation is that the conversion of part of the carbon monoxide (CO) in the syngas feed to carbon dioxide ($CO_2$), driven by catalysts which are active to the water-gas-shift reaction, contributes noticeably to the aforementioned decrease in the selectivity and time-yield to higher alcohols. Moreover, the by-production of $CO_2$ penalizes the efficiency of the process by increasing costs of separation and recycling, particularly so for processes transforming syngas feeds with $H_2$/CO ratios close to or higher than 2.0, and which hence do not benefit from the additional production of hydrogen at the expenses of CO consumption via the WGSR.

A further technical limitation of the state of the art as found by the inventors is the fact that the remarkably different temperature operation ranges of the process of iron-catalyzed Fischer-Tropsch synthesis of $C_{2+}$ olefins from syngas, and the process of olefin hydroformylation to alcohols, precludes the integration of these two processes in a single reactor to convert syngas to higher alcohols in a single reactive step.

The inventors further considered that it is advantageous to provide a process which allows circumventing these limitations of the state-of-the-art, thus enabling the production of higher alcohols with high selectivity at high CO conversion levels and low by-production of $CO_2$ from syngas -including hydrogen-rich syngas streams characterized by a molar $H_2$/CO ratio equal or higher than 1.7- in a single reactive step.

Therefore, it is one object of the present invention to provide an improved process for converting synthesis gas into $C_{3+}$ alcohols, i.e. alcohols having 3 to 12 or more carbon atoms in their aliphatic hydrocarbon structure, with high alcohol selectivity at high CO conversion levels in a single reactive step.

It has been found by the inventors that such a process can be implemented by:

(1) contacting a feed stream comprising syngas with:

(i) a catalyst which is active for the Fischer-Tropsch synthesis, essentially inactive for the water-gas-shit reaction, and selective for the production of $C_{2+}$ terminal olefins;
(ii) a catalyst which is stable and active for the reductive hydroformylation of olefins, and it is highly selective for the production of terminal alcohols;
(iii) a solvent medium which contains both the Fischer-Tropsch and the hydroformylation catalysts;

whereby the two reactions are operated concomitantly, in the same reactor, i.e. a vessel where one or various chemical reactions are conducted, and under a single suitable set of reaction conditions; and
(2) recovering higher alcohols from the reaction products.

In more detail, the present invention refers to a process for converting a syngas feed stream into ($C_{3+}$) alcohols

wherein,

in a first step, a syngas feed stream comprising carbon monoxide and hydrogen with a $H_2/CO$ ratio in the range of 0.4 to 4.0 is contacted, in a single reactor, in the presence of a solvent in a temperature range of 298 K to 533 K, preferably 373 K to 493 K, under a reaction pressure of 1 bar to 300 bar, preferably 80 bar to 200 bar, with a catalyst combination comprising

    (i) a Fischer-Tropsch catalyst as a first catalyst, which converts syngas to a hydrocarbon mixture comprising $C_{2+}$ olefins and which is essentially inactive for the water-gas-shift reaction and,
    (ii) a hydroformylation catalyst as a second catalyst, which is stable and active for the reductive hydroformylation of olefins and which is highly selective for the production of terminal alcohols;

in a ratio between the Fischer-Tropsch catalyst and the hydroformylation catalyst, expressed as the molar ratio of the metal in the Fischer-Tropsch catalyst and the metal in the hydroformylation catalyst is selected in the range of 30:1-1:4, more preferably in the range of 10:1-1:2, and most preferably in the range of 4:1-1:1,and,

in a second step, $C_{3+}$ alcohols formed as reaction products are recovered from the reactor.

Thus, the present invention accordingly provides, in a first aspect, a process by means of which a syngas feed including syngas feed streams with $H_2/CO$ ratios preferably equal or higher than 0.7 is converted into $C_{3+}$ alcohols with high alcohol selectivity at high CO conversion levels in a single step by the combination of a Fischer-Tropsch catalyst and an olefin reductive hydroformylation catalyst in a single reactor. The present invention additionally provides a Fischer-Tropsch catalyst which can be applied in said process.

The present invention further provides a process to produce a mixture of higher alcohols characterized by a high chain-growth probability, as determined in the carbon chain length ($n$) range from 3 to 8 carbon atoms, equal to or greater than 0.60, preferably equal to or greater than 0.65, and more preferably equal to or greater than 0.70, and thus said alcohols spanning from $C_3$ to $C_{12+}$ alcohols, and a process with a minimum selectivity to $CO_2$.

The present invention also provides a Fischer-Tropsch catalyst with high selectivity to $C_{2+}$ alpha-olefins and low selectivity to $CO_2$, which can be applied in said process.

Thus in a second aspect, the present invention is directed to the catalyst combination for converting a syngas feed gas into $C_{3+}$ alcohols comprising

    (i) a Fischer-Tropsch catalyst as a first catalyst, which converts syngas to a hydrocarbon mixture comprising $C_{2+}$ olefins and which is essentially inactive for the water-gas-shit reaction and,
    (ii) a hydroformylation catalyst as a second catalyst, which is stable and active for the reductive hydroformylation of olefins and which is highly selective for the production of terminal alcohols;

in a ratio between the Fischer-Tropsch catalyst and the hydroformylation catalyst, expressed as the molar ratio of the metal in the Fischer-Tropsch catalyst and the metal in the hydroformylation catalyst is selected in the range of 30:1-1:4, more preferably in the range of 10:1-1:2, and most preferably in the range of 4:1-1:1.

In a third aspect, the present invention is directed to the use of a catalyst combination as defined before in a single-pot process for converting a syngas feed stream into ($C_{3+}$) alcohols.

As stated before, the present invention is concerned, in a first aspect, with a process to convert a feed gas stream comprising synthesis gas into higher ($C_{3+}$) alcohols by means of the combination of a Fischer-Tropsch catalyst and an olefin hydroformylation catalyst. In a second aspect, the invention relates to a Fischer-Tropsch catalyst suitable to be applied in said process.

The term "selectivity" as used in the context of the present invention is defined on a carbon basis, and it is therefore understood to mean the ratio between the total molar amount of carbon in the products of interest - higher alcohols in this case- and the total molar amount of carbon in all reaction products.

The term "conversion" as used in the context of the present invention is expressed as a percentage and understood to mean the fraction of carbon monoxide, or alternatively carbon monoxide and carbon dioxide, in the syngas feed which is converted in the reactor to other carbon-containing compounds.

The term "time-yield" as used in the context of the present invention is understood to mean the total amount of the products of interest - higher alcohols in this case- produced per unit mass of catalyst and time.

The term "molar abundance" as used in the context of the present invention is understood to mean the mole-based fraction of any given compound or family of compounds, for instance alpha-olefins, within the total or a fraction of the products of a reactive process, for instance $C_3$-$C_{10}$ hydrocarbons, expressed on a per 100 (%) basis.

It has been deemed essential for the process of this invention that a Fischer-Tropsch catalyst which converts syngas to a hydrocarbon mixture enriched in $C_{3+}$ alpha olefins, and shows limited activity for the WGSR, is applied to convert a syngas feed stream in the presence of a hydroformylation catalyst which is stable and active under the conditions of the process and catalyzes the reductive hydroformylation of linear olefins to alcohols.

A first embodiment of the present invention refers to a process which comprises:

(1) to bring in contact a feed stream comprising syngas with:

(i) a first catalyst, the Fischer-Tropsch catalyst, which converts syngas to a hydrocarbon mixture comprising $C_{2+}$ olefins and it is essentially inactive for the water-gas-shift reaction and;
(ii) a second catalyst, the hydroformylation catalyst, which is stable and active for the reductive hydroformylation of olefins, and it is highly selective for the production of terminal alcohols;
(iii) a solvent medium which contains and surrounds both the Fischer-Tropsch and the hydroformylation catalysts;

whereby the two reactions are operated concomitantly, in the same reactor and under a single suitable set of reaction conditions; and
(2) to recover higher alcohols from the reaction products.

It has been deemed important for the process of the invention that the Fischer-Tropsch catalyst is active for the Fischer-Tropsch synthesis and essentially inactive for the WGSR and it provides high selectivity to $C_{2+}$ alpha-olefin hydrocarbons at mild reaction temperatures. It has also been deemed important for the process of the invention that the hydroformylation catalyst is chemically stable, active for the reductive hydroformylation of olefins and highly selective to terminal alcohols in the same temperature range. It has also been deemed important that both catalysts are active for their own reaction when coexisting in a common fluid medium under a common set of reaction conditions, i.e. reaction temperature and pressure.

A Fischer-Tropsch catalyst suitable for the process of the current invention should be active for the Fischer-Tropsch synthesis of hydrocarbons from syngas under standard Fischer-Tropsch synthesis conditions. Preferably this activity, expressed as a metal mass-specific rate of CO conversion, is equal or higher than 5 $mmol_{CO}$ $g_{metal}^{-1}$ $h^{-1}$, when a syngas feedstock with a $H_2$/CO molar ratio of 2.0 is converted on the catalyst at a reaction temperature equal to or lower than 483 K and a reaction pressure equal to or greater than 15 bar. A high specific activity is desired to achieve higher CO conversion and product time-yields in the process of the invention. Preferably, the metal mass-specific rate of CO conversion is higher than 15 mmolco $g_{metal}^{-1}$ $h^{-1}$, and yet more preferably the metal mass-specific rate of CO conversion is higher than 100 mmolco $g_{metal}^{-1}$ $h^{-1}$.

Preferably, the Fischer-Tropsch catalyst suitable for the process of the current invention should display a limited activity for the water-gas-shift reaction, hence the selectivity to $CO_2$ preferably does not exceed 5%, when a syngas feedstock with a $H_2$/CO molar ratio of 2.0 is converted on the catalyst at a reaction temperature equal to or lower than 483 K, a reaction pressure equal to or greater than 15 bar and a CO conversion equal to or greater than 15% is achieved. More preferably the selectivity to $CO_2$ does not exceed 2%. A very low production of $CO_2$ is desirable to maximize the efficiency of the CO conversion process and avoid the need for large recycle streams and additional reverse-water-gas-shift unitary operations, hence contributing to process intensification, a lower overall energy requirement and a lower overall carbon footprint of the process.

It is important for the process of the present invention that the Fischer-Tropsch catalyst is capable to convert a syngas feed stream with high selectivity to $C_{3+}$ alpha-olefins and low selectivity to $CO_2$. Without wishing to be bound by any theory, the inventors believe that a high fraction of $C_{3+}$ alpha-olefins within the hydrocarbons produced by the Fischer-Tropsch catalyst under the process conditions is important to achieve a high rate for the olefin hydroformylation reaction and a high selectivity to higher alcohols within the products of the inventive process.

The performance of a given Fischer-Tropsch catalyst can be adequately determined by means of catalytic tests in which the Fischer-Tropsch catalyst is tested alone, without any additional catalyst, for the gas-phase conversion of a syngas feed stream under reaction conditions which are standard for the industrial operation for the Fischer-Tropsch synthesis, and the corresponding product stream analyzed, for instance by gas chromatography analytical methods.

Preferably, the Fischer-Tropsch catalyst suitable for the process of the current invention should additionally display an unusually high selectivity to $C_{3+}$ alpha-olefin products under standard Fischer-Tropsch reaction conditions. In one embodiment, the molar abundance of alpha-olefin hydrocarbons in the hydrocarbon products with hydrocarbon chain lengths in the range of $C_3$-$C_{10}$ is equal to or higher than 30%, when a syngas feedstock with a $H_2$/CO molar ratio of 2.0 is converted on the catalyst at a reaction temperature equal to or lower than 483 K, a reaction pressure equal to or greater than 15 bar and a CO conversion equal to or greater than 15% is achieved. More preferably, the molar abundance of alpha-olefin hydrocarbons in the hydrocarbon products with hydrocarbon chain lengths in the range of $C_3$-$C_{10}$ is equal to or higher than 40%. Most preferably, the molar abundance of alpha-olefin hydrocarbons in the hydrocarbon products with hydrocarbon chain lengths in the range of $C_3$-$C_{10}$ is equal to or higher than 50%.

Given that alpha-olefin hydrocarbons are considered to be primary reaction products of the Fischer-Tropsch synthesis, and without wishing to be bound by any theory, the inventors have considered that a particularly suitable Fischer-Tropsch catalyst for the process of the invention shows a limited activity for secondary reactions of alpha-olefin hydrocarbons, particularly hydrogenation and double-bond isomerization, which contribute to lowering the abundance of alpha-olefin compounds within the hydrocarbon products in standard Fischer-Tropsch synthesis processes.

As stated above, the process of the invention is characterized by using a combination of a Fischer-Tropsch catalyst which is active for the Fischer-Tropsch synthesis and essentially inactive for the WGSR and which provides high selectivity to $C_{2+}$ alpha-olefin hydrocarbons at mild reaction temperatures, and a hydroformylation catalyst which is chemically stable, active for the reductive hydroformylation of olefins and highly selective to terminal alcohols in the same temperature range.

Thus, in accordance with the invention, the Fischer-Tropsch catalyst is a supported metal catalyst. The concept of "supported metal catalyst" is understood in the art as a catalyst composition comprising the catalytically active metal part, dispersed in the form of particles with an average diameter typically smaller than 100 nm, which is therefore active, or can be converted into an active phase in situ prior to or during the usage of the catalyst, and a catalytically non-active part, wherein the catalytically non-active part, denoted as carrier or support material in the art, is generally porous and forms the majority of the catalyst on a mass basis.

Several methods are known in the art to incorporate the active metal part on a support material. These methods comprise preparation techniques such as impregnation, deposition precipitation, ion exchange, electrochemical deposition, electrostatic adsorption, co-precipitation which are known methods in the art. The documents "Handbook of Heterogeneous Catalysis", G. Ertl, H. Knözinger, F. Schüth, J. Weitkamp (Editors); Volume 1, Wiley-VCH Verlag GmbH & Co. Weinheim, 2008 and "Synthesis of solid catalysts", K. P. de Jong (Editor); Wiley-VCH Verlag GmbH & Co. Weinheim, 2009 provide good references for the existing methods to incorporate the active metal part on a support material.

The porous support material should be chemically stable under the reaction conditions encountered in a process of syngas conversion into hydrocarbons. Said porous carrier can be an oxide selected among those oxide porous carriers which are employed as support materials for supported metal catalysts in the art, and which can comprise, but it is not limited to, $Al_2O_3$, $SiO_2$, $TiO_2$, or combinations thereof. Alternatively, the support material can be based on a carbide or oxy-carbide material such as SiC, $SiO_xC_y$, with x being in the range of 0<x<2 and y in the range of 0<y<1. Alternatively, the support material can be based on carbon. Alternatively, the support material can be a composite incorporating a combination of said materials.

The porosity of the Fischer-Tropsch catalyst becomes dictated primarily by the support material, the contribution of the active metal component to the total porosity being limited, aside from the corresponding mass dilution effect on mass-specific porosity parameters, i.e. those expressed per unit total mass of catalyst. The porosity of a solid material can be determined in the art by analytical methods such as nitrogen physisorption combined to suitable mathematical models for the analysis of the physisorption isotherms, thermoporometry combined to suitable mathematical models for the analysis of the corresponding scanning calorimetry profiles, mercury intrusion porosimetry combined with suitable mathematical models for the analysis of the intrusion isotherms or by electron microscopy methods such as transmission electron microscopy, scanning electron microscopy and their tomographic variations which allow imaging in the three directions in space, combined with suitable methods for quantification of said porosity. The pore volume and total pore size distribution for the solid material might be derived from these methods. In the context of this invention, the term "total pore volume" is understood to mean the volume of all the pores in the material, expressed on a per material mass basis. In the context of this invention, the term "pore size distribution" is understood to mean the evolution of the pore volume fraction, relative to the total pore volume, as a function of the pore diameter. According to the International Union of Pure and Applied Chemistry (IUPAC), pores are denoted micropores when their diameter is smaller to 2 nm, they are denoted mesopores if their diameter is in the range of 2-50 nm and they are denoted macropores if their diameter is larger than 50 nm.

In a preferred embodiment of the present invention, the support material of the Fischer-Tropsch catalyst contains mesopores and macropores. Preferably, the volume of macropores accounts for at least a 5 Vol.-% of the total pore volume, and more preferably for at least a 20 Vol.-% of the total pore volume, the remainder being mesopores.

In a preferred embodiment of the invention, the Fischer-Tropsch catalyst comprises, as active metal, Co, Ru or any combination thereof. More preferably, Co accounts for more than 90% of the active metal. Yet more preferably, Co accounts for more than 98% of the active metal.

Preferably, the weight loading of active metal, i.e. the weight fraction of the total catalyst which corresponds to the active metal in said catalyst, is in the range of 1-50 wt%, and more preferably in the range of 10-35 wt%.

Metals are incorporated on the porous support using metal precursor compounds. Examples of cobalt containing precursors are inorganic and organic cobalt salts, cobalt clusters, and cobalt organometallic complexes. Representative of these compounds are cobalt nitrate, cobalt chloride, cobalt sulphate, cobalt phosphate, cobalt chloroplatinate, cobalt hydroxide, cobalt acetate, cobalt acetylacetonate, cobalt oxalate, cobalt oleate, cobalt citrate, cobalt carbonyl, and the like. Cobalt precursors can provide cobalt in a zero oxidation state, II oxidation state, III oxidation state, or a combination thereof. According to a preferred embodiment of the invention, the Fischer-Tropsch catalyst is prepared using cobalt nitrate as cobalt precursor. Examples of ruthenium containing precursors are inorganic and organic ruthenium salts, ruthenium clusters, and ruthenium organometallic complexes. Representative of these compounds are ruthenium acetylacetonate, ruthenium chloride, ruthenium nitrosyl nitrate, triruthenium dodecacarbonyl, bis(cyclopentadienyl)ruthenium(II), and the like. Ruthenium precursors can provide ruthenium in a zero oxidation state, II oxidation state, III oxidation state, or a combination thereof. According to a preferred embodiment of the invention, the Fischer-Tropsch catalyst is prepared using ruthenium nitrosyl nitrate as ruthenium precursor.

It is customary in the art, that supported metal catalysts comprise so-called promoters, in addition to the main active metal part and the support material. In general, a promoter is a substance that enhances the catalyst's performance in terms of any of activity, selectivity to a given product or fraction of products, stability under the operation conditions, or combinations thereof.

In one preferred embodiment of the invention when a Co species is used, the Fischer-Tropsch catalyst comprises at least one metal promoter which assists the reduction of cobalt species into its catalytically active metallic state and which is selected from the list of ruthenium, rhenium, platinum, palladium, silver, gold, copper, preferably from the list of ruthenium, rhenium, platinum, palladium, and more preferably it is ruthenium. The weight content of this promoter is preferably lower than 10 wt%, on the basis of the total mass of catalyst, and more preferably lower than 2 wt%.

In one embodiment of the invention, the Fischer-Tropsch catalyst comprises at least a second promoter. Said promoter can be selected from the list of alkali metals, alkaline earth metals, lanthanides, transition metals, boron, aluminium, gallium, indium, carbon, silicon, germanium, tin, nitrogen, phosphorous, arsenic, antimony, and any combination thereof. Any of these elements can be either in elementary form or in ionic form. In a preferred embodiment of the invention, the promoter contributes to a higher selectivity to olefin Fischer-Tropsch products under standard reaction conditions for the Fischer-Tropsch synthesis. In a preferred embodiment of the invention, said second promoter is selected from the list of alkali metals, alkaline earth metals, lanthanides, yttrium, manganese, zinc, gallium, boron, indium, and any combination thereof. In a yet more preferred embodiment of the invention, said second promoter is selected from the list of alkali metals, alkaline earth metals, lanthanides, yttrium and manganese. Said second promoter is present in a mass larger than 0 wt% and lower than 50 wt%, more preferably larger than 0 wt% and lower than 20 wt%, and yet more preferably larger than 0 wt% and lower than 10 wt%, calculated on the basis of the total mass of the Fischer-Tropsch catalyst.

Prior to its use in catalysis, the Fischer-Tropsch catalyst is typically subjected to thermal treatments in the presence of hydrogen or an alternative reducing agent to render part or the total of the active metal part into the metallic state. In a preferred embodiment, said reduction treatment takes place at a temperature in the range of 473-873 K, and more preferably in the range of 573-773 K in flow of a stream comprising hydrogen, either as pure gas or in combination with an inert gas from the list of nitrogen, helium, argon.

As the second catalyst besides the Fischer-Tropsch catalyst, the hydroformylation catalyst is used in the inventive process.

Said hydroformylation catalyst is characterized by a combination of at least:

(i) at least one metal precursor compound; and
(ii) at least one organic or inorganic ligand, to form a coordination complex.

The term "ligand" as used in the context of the present invention is understood as an organic or inorganic ion or molecule which is bound to one or more central metal atoms via a coordinative bond. The process of this type of bonding is known as complexation and the resulting aggregate is referred to as coordination complex or simply complex.

The metal in the hydroformylation catalyst can be selected from the list of cobalt, ruthenium, rhodium, iridium, or any combination thereof. In a preferred embodiment, the metal is selected from the list of cobalt, ruthenium or combinations thereof. In a yet more preferred embodiment, the metal is cobalt.

In a preferred embodiment of the present invention, the metal precursor compound is selected from the list consisting of cobalt carbonyl complexes such as cobalt-tetracarbonyl hydride, dicobalt-octacarbonyl, and carbonyl clusters; and cobalt salts as formate, acetate, acetylacetonate, carboxylate, carbonate, oxalate, halide, amine, imide, and any combination thereof. In a yet more preferred embodiment of the present invention, the metal precursor compound is selected from the list consisting of cobalt carbonyl complexes such as cobalt-tetracarbonyl hydride, dicobalt-octacarbonyl, and cobalt carbonyl clusters.

The organic ligand can be selected from the list consisting of oxygen-containing ligands, phosphorus-containing ligands, nitrogen-containing ligands, arsenic-containing ligands and combinations thereof. More particularly, the oxygen-containing ligands are selected from the list consisting of alcohols, ethers, ketones, acetals and combinations thereof. Non-limiting examples that can be cited are 2-propanol, phenol, dibutylether, diphenylether, 1,4-dioxane, 1,3-dioxolane, diglyme, acetone, methylethylketone, acetophenone, 2,2-dimethoxypropane and 2,2-di(2-ethylhexyloxy)-propane. More particularly, the nitrogen-containing ligands are selected from the list consisting of monoamines, di-, tri- and polyamines, imines, diimines, pyridines, bipyridines, imidazoles, pyrroles and pyrazoles. Non-limiting examples that can be cited are trimethylamine, triethylamine, ethylenediamine, quinuclidine, diisopropylethylamine, N,N-dimethylbenzylamine, *N,N*-dimethylaniline, *N,N*-diisopropylaniline, diethylenetriamine, diazabicyclooctane, N,N'-dimethylethane-1,2-diimine, N,N'-di-t-butylethane-1,2-diimine, N,N'-di-t-butylbutane-2,3-diimine, N,N'-diphenylethane-1,2-diimine, N,N'-bis-(2,6-dimethylphenyl)ethane-1,2-diimine, N,N'-bis-(2,6-diisopropylphenyl)ethane-1,2-diimine, N,N'-diphenylbutane-2,3-diimine, N,N'-bis-(2,6-dimethylphenyl)butane-2,3-diimine, N,N'-bis-(2,6-diisopropylphenyl)butane-2,3-diimine, pyridine, t-butyl-2-pyridine, di-(t-butyl)-2,6-pyridine, 2,2'-bipyridine, imidazole, N-methylimidazole, N-butylimidazole, pyrrole, N-methylpyrrole and 2,5-dimethylpyrrole.. More particularly, the phosphorus-containing ligands are selected from the list consisting of phosphines, polyphosphines, phosphine oxides and phosphites. Non-limiting examples that can be cited are triethylphosphine, tributylphosphine, trihexylphosphine, trioctylphosphine, triisopropylphosphine, tricyclohexylphosphine, tricyclopentylphosphine, hexylphobane, cyclohexylphobane, triphenylphosphine, diethylphenylphosphine, ethyldiphenylphosphine, bis(diphenylphosphino)ethane, bis(diphenylphosphino)butane, and triphenylphosphite. In a preferred embodiment of the invention, the ligand is selected from the list consisting of phosphorus-containing ligands. In yet a more preferred embodiment of the invention, the ligand is selected from alkyl-phosphines, aryl-phosphines or any combination thereof.

Without wishing to be bound by any particular theory, the hydroformylation catalyst can be considered to be present in the form of a coordination complex under the conditions of pressurized synthesis gas atmosphere as that pertaining to the inventive process. Such coordination complex might have the chemical formula $H_x(M)_y(CO)_z(L)_n$, where $x$ is equal to or greater than zero, $y$ is equal to or greater than 1, $z$ is equal to or greater than zero and $n$ is equal to or greater than zero, and M denotes a metal, and L denotes an organic ligand.

The catalytic composition of the hydroformylation catalyst is obtained by mixing the metal precursor compound and the ligand in a solvent medium. It is also possible to mix pre-existing individual solutions of the metal precursor compound and the ligand.

In one embodiment of the invention, the metal precursor compound and the ligand, or a suitable precursor thereof, are mixed in a solvent medium where the reaction of syngas conversion of the inventive process will take place, directly prior to the start of the syngas conversion reaction and in the presence of the Fischer-Tropsch catayst. In another embodiment of the invention, the ligand, or a precursor thereof, is fed into the reactor over the course of the reaction of syngas conversion. In a preferred embodiment of the invention, a coordination complex comprising the metal and the ligand is prepared in advance of the commencement of the syngas conversion reaction, in the absence of the Fischer-Tropsch catalyst, by mixing the metal precursor compound with the ligand, or a suitable precursor thereof, in a solvent. Without wishing to be bound by any particular theory, the preparation of a coordination complex, in advance of the commencement of the syngas conversion reaction, in the absence of the Fischer-Tropsch catalyst, might ensure the coordination of the ligand to the metal provided with the metal precursor compound, reducing the amount of unbound ligand at the time the hydroformylation catalyst and the Fischer-Tropsch catalyst are integrated in the same reaction medium, and thus the propensity for ligand to coordinate to metal species from the Fischer-Tropsch catalyst leading to metal leaching into solution. In a preferred embodiment of the invention, the preparation of said coordination complex prior to the commencement of the syngas conversion reaction is performed by exposing a solution of the metal precursor compound with the ligand, or a suitable precursor thereof, to a gas comprising carbon monoxide or syngas. The temperature for this treatment might be selected in the range of 298 K to 533 K. Preferably, the reaction temperature is selected in the range of 373 K to 493 K. The total pressure for this treatment might be selected in the range of 1 to 300 bar, preferably in the range of 80-200 bar.

The preparation of the hydroformylation catalyst in the form of a coordination complex prior to or during the syngas conversion reaction is a preferred embodiment, however, the use of metal salts which already comprise the organic ligand is possible, and it does not limit the invention.

According to the inventive process, the molar ratio ligand/metal in the formulation of the hydroformylation catalyst is greater than 0.1. Without wishing to be bound by any particular theory, a high concentration of ligand in the reaction medium might lead to metal leaching from the surface of the Fischer-Tropsch catalyst due to the formation of stable coordination complexes. Preferably, the molar ratio ligand/metal in the formulation of the hydroformylation catalyst is selected in the range of 1-5. Most preferably, said molar ratio is selected in the range of 1-3.

The ratio between the Fischer-Tropsch catalyst and the hydroformylation catalyst has been deemed important for the inventive process. This ratio, as used in the context of the present invention, is expressed as the ratio between the total metal in the Fischer-Tropsch catalyst and the total metal in the hydroformylation catalyst, on a molar basis, present inside the reactor of the inventive process. In one embodiment of the present invention, the ratio between the Fischer-Tropsch catalyst and the hydroformylation catalyst, expressed as the molar ratio of cobalt (Co) in the Fischer-Tropsch (FT) catalyst and cobalt in the hydroformylation (HF) catalyst (Co(FT):Co(HF)), is selected in the range of 30:1-1:4. More preferably, said catalyst ratio is selected in the range of 10:1-1:2. Most preferably, said catalyst ratio is selected in the range of 4:1-1:1.

According to the process of the present invention, the Fischer-Tropsch catalyst, the hydroformylation catalyst and the solvent are added into a single reactor, to which the syngas feed stream is fed.

In one embodiment of the invention, the reactor is operated in a continuous mode, whereby at least one stream

EP 4 112 169 A1

of the gas feed and one stream of the solvent are provided continuously to the reactor inlet and a product stream is continuously removed at the outlet of the reactor.

In another embodiment of the invention, the reactor is operated in a discontinuous (batch-type) mode, whereby the syngas feed, the solvent, the Fischer-Tropsch catalyst and the hydroformylation catalyst are provided into the reactor prior to the reactor temperature being set to the reaction temperature, the reaction proceeds without additional streams being provided to the reactor inlet during the reaction. After a certain reaction time the reaction is terminated by lowering the temperature below the range of temperatures for the inventive process, and at least one product stream is collected from the reactor outlet.

In another embodiment of the invention, the reactor is operated in a semi-continuous mode, whereby the syngas feed, the solvent, the Fischer-Tropsch catalyst and the hydroformylation catalyst are provided into the reactor prior to the reactor temperature being set to the reaction temperature, the reaction proceeds under continuous provision of a syngas feed stream to the reactor inlet during the progress of the reaction, the reaction is terminated after a certain reaction time by lowering the temperature below the range of temperatures for the inventive process, and at least one product stream is collected from the reactor outlet.

The solvent used in the process of the present invention is not particularly limited as long as it is thermally stable at the temperature applied in the inventive processes and does not undergo side reactions with neither the intermediate aldehyde products nor the final alcohol products of the reductive hydroformylation reaction. The solvent might be selected from the list of aliphatic hydrocarbons, aromatic hydrocarbons, oxygenated compounds, water, nitrogenated compounds, halogenated organic compounds, liquid organic salts and combinations thereof. More particularly, aliphatic hydrocarbon solvents are selected from the non-limiting list consisting of n-hexane, n-decane, n-dodecane, n-hexadecane, iso-pentane (2-methylbutane), iso-hexane (2-methylpentane), iso-octane (2,2,4-Trimethylpentane) and 2,6,11,15-tetramethylhexadecane. Again more particularly, aromatic hydrocarbon solvents are selected from the non-limiting list consisting of benzene, toluene, xylene, trimethylbenzenes and naphthalene. Again more particularly, oxygenated compound solvents are selected from the non-limiting list consisting of dimethylformamide, tetrahydrofurane, di-n-butylether, cyclohexanone, methylisobutylketone, ethyleneglycole, propyleneglycole, cyclopropylcarbonate, glycerole and ethylacetate. Again more particularly, nitrogenated compound solvents are selected from the non-limiting list consisting of acetonitrile, benzonitrile, diethanolamine, tri-n-butylamine, triethylamine, N,N-dimethylaniline, pyridine, 2-methylpyridine, N-methylmorpholine, N-methylpiperidine, N-methypyrolle and quinoline. Again more particularly, halogenated compound solvents are selected from the non-limiting list consisting of methylenechloride, chloroform, 1,2-dichloroethane, perfluoropentane, perfluorohexane, perfluorotoluene, mono-fluorobenzene and perfluorobenzene. Again more particularly, liquid organic salt solvents are selected from the non-limiting list consisting of salts comprising of organic cations of the type imidazolium, pyridinium, 1,2,3-triazolium, morpholinium, piperidinium, pyrrolidinium, pyrazolium, ammonium and phosphonium associated with anions of the type $PF_6^-$, $BF_4^-$, $NTf^{2-}$ (bis(trifluoromethylsulfonyl)amide), OTf$^-$ (trifluoromethylsulfonate), DCA$^-$ (dicyanamide), acetate and $CH_3SO_3^{3-}$.

In a preferred embodiment of the invention, the syngas contact time with the catalysts is selected to achieve a CO conversion in the range of 5-100%, preferably in the range from 20-80% and most preferably in the range of 40-80%.

According to the process of the invention, the syngas mixture applied as feed has a molar $H_2/CO$ ratio in the range of 0.4-4.0. In a preferred embodiment, the syngas mixture applied as feed has a molar $H_2/CO$ ratio in the range of 1.0-2.5, and more preferably it is in the range of 1.5 to 2.0.

According to the process of the invention, the reaction pressure can be selected in the range of 50 to 300 bar, preferably in the range of 80-200 bar.

According to the process of the present invention, the reaction temperature can be selected in the range of 373 K to 533 K. More preferably, the reaction temperature can be selected in the range of 423 K to 493 K. In a preferred embodiment of the invention, the reaction temperature is higher than the critical temperature and the reaction pressure is higher than the critical pressure of at least one compound comprised in the solvent. The term "critical point" as used in the context of the present invention is understood to mean the combination of temperature (critical temperature) and pressure (critical pressure) on a temperature vs. pressure phase diagram for a compound at which both the liquid and gas phases of said compound have the same density, and are therefore indistinguishable. At pressures and temperatures higher than the critical point, the substance is in its supercritical state.

Particularly none of the prior art references achieves the combined results displayed by the process of the invention, such as a high selectivity to $C_{3+}$ higher alcohols (let alone higher than 40%), and low selectivity to $CO_2$ (let alone lower than 2%) in conjunction with CO conversions higher than 45%.

The present invention will now be described in more detail by means of the following Figures and Experimental Part.

In the Figures:

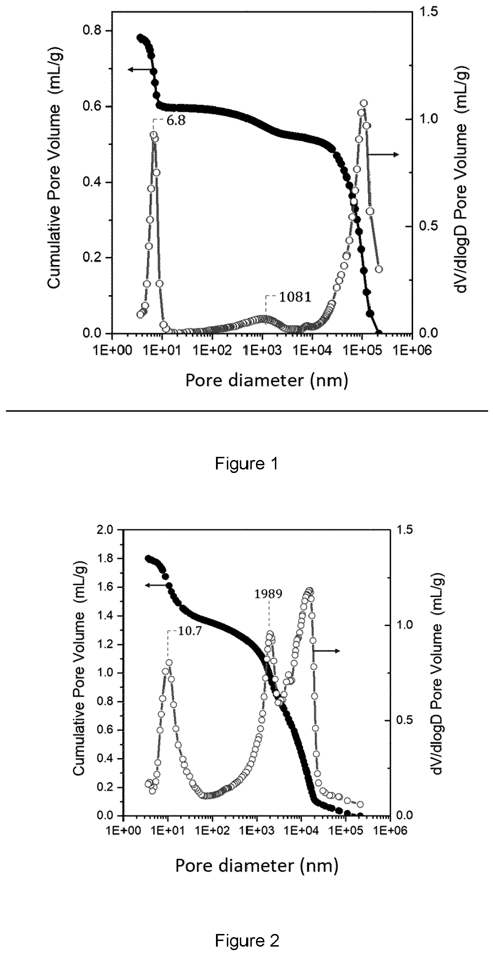

Figure 1 shows the cumulative intrusion isotherm (full symbols, left y-axis) and the pore size distribution (open symbols, right axis) as determined by mercury intrusion porosimetry for catalyst CoRu/Al$_2$O$_3$m_. The abscissa x-

11

axis reports the pore size in nanometers, increasing from left to right and with a 10-logarithmic scale.. The left ordinates y-axis reports the cumulative mercury intrusion volume, increasing from bottom to top, with a linear scale. The right y-axis reports the log differential pore volume, increasing from bottom to top, with a linear scale, and Figure 2 shows the cumulative intrusion profile (full symbols, left y-axis) and the pore size distribution (open symbols, right axis) as determined by mercury intrusion porosimetry for catalyst CoRu/Al$_2$O$_3$mM. The abscissa x-axis reports the pore size in nanometers, increasing from left to right and with a 10-logarithmic scale. The left ordinates y-axis reports the cumulative mercury intrusion volume, increasing from bottom to top, with a linear scale. The right y-axis reports the log differential pore volume, increasing from bottom to top, with a linear scale. In both Figure 1 and Figure 2, peaks in pore size distribution at pore diameters higher than 10000 nm correspond to voids between the catalyst particles.

## Experimental Part

## Methods for determining the properties of the materials

The following methods were employed for determining the properties of materials as prepared and used in the Examples listed herein below:
Macropore and mesopore size distributions, as well as total macropore volumes of solid materials were determined by means of mercury intrusion porosimetry in a Micromeritics AutoPore IV 951 apparatus. In a typical experiment, 80-150 mg of sample (< 0.08 mm) were dried at 383 K for 72 h before the measurement. The intrusion-extrusion isotherms were recorded at room temperature in the pressure range of 1.01-59848 psia with an equilibration rate of 0.1 $\mu$L g$^{-1}$ s$^{-1}$. For the determination of pore diameter and volume, a cylindrical pore model was considered, with a Hg density of 13.55 g cm$^{-3}$ and a contact angle of 141°. Pore size distributions were visualized by plotting the logarithmic differential volume intrusion (dV/dlogD) versus the equivalent pore diameter expressed in nanometers.

Specific surface area of solid materials was determined by means of nitrogen physisorption. Nitrogen physisorption isotherms were recorded using a Micromeritics ASAP instrument after degassing the sample (ca. 100 mg, < 0.08 mm particle size) at 523 K under vacuum for 10 h. Surface areas were derived using the B.E.T method in the relative pressure (P/P$_0$) regime of 0.05-0.30. Total mesopore volumes were based on the amount of N$_2$ adsorbed at a relative pressure P/P$_0$=0.95.

The macroscopic particle size of solid materials was adjusted and determined using calibrated Retsch stainless steel sieves.

The cobalt metal loading in cobalt-based Fischer-Tropsch catalysts was determined by means of hydrogen temperature-programmed reduction (H$_2$-TPR) in a Micromeritics Autochem 2910 device. In a typical experiment, about 100 mg of sample were initially flushed with Ar flow (50 cm$^3$ min$^{-1}$) at room temperature for 30 min, then the gas was switched to 10 vol% H$_2$ in argon (Ar) and the temperature increased up to 1123 K at a heating rate of 10 K min$^{-1}$. A downstream 2-propanol/dry ice trap was used to retain the water generated during the reduction. The H$_2$ consumption rate was monitored in a thermal conductivity detector (TCD) previously calibrated via the injection of known volumes of hydrogen using a gas syringe. The cobalt loading was determined from the total hydrogen consumption assuming all cobalt to be present as Co$_3$O$_4$ in the calcined catalysts, i.e. prior to the H$_2$-TPR experiment, and full reduction to metallic cobalt, which results in a reduction stoichiometric H$_2$/Co molar ratio of 4/3. The hydrogen consumption associated to the Ru promoter was considered negligible. The cobalt metal content in the cobalt-based hydroformylation catalyst was determined by direct weighing of the corresponding cobalt precursor of known molecular formula.

## Synthesis of catalysts according to the current invention

## Synthesis of a Fischer-Tropsch catalyst according to the invention (CoRu/Al$_2$O$_3$_m)

An Al$_2$O$_3$ support material with a pore size distribution with a major contribution in the mesopore range (peak at 6.8 nm, Figure 1), and a minor contribution in the macropore range (peak at 1081 nm, Figure 1) denoted hereafter as Al$_2$O$_3$_m, was prepared by submitting commercial powder pseudo-boehmite (Catapal B, from Sasol Materials) to a thermal treatment at 823 K for 5 hours in a muffle oven under stagnant air atmosphere. The resulting Al$_2$O$_3$ was pressed into self-supported wafers, crushed and sieved to retain particles in the size range of < 0.08 mm. To synthesize the Fischer-Tropsch catalyst, the Al$_2$O$_3$ particles were first dried under dynamic vacuum (rotatory pump) at 423 K for 2 hours. Subsequently, the dry solid was impregnated under static vacuum with an aqueous solution containing Co(NO$_3$)$_2$·6H$_2$O (1.5 M, Sigma-Aldrich, ≥98%, CAS: 10026-22-9) and ruthenium (III) nitrosyl nitrate (in dilute nitric acid, Sigma-Aldrich, CAS: 34513-98-9), previously prepared to have an atomic ratio Ru/Co=0.007, and further acidified with 0.25 vol% HNO$_3$ (69-70 vol.% in H$_2$O, J.T. Baker, ≥99%, CAS: 7697-37-2). The volume of solution infiltrated in the Al$_2$O$_3$ support was equivalent to 90% of the total mesopore volume of the support as determined by N$_2$ physisorption. After impregnation,

the solid was dried in the form of a packed bed in a tubular reactor at 343 K under vertical downward $N_2$ flow (200 $cm^3$ $g_{cat}^{-1}$ $min^{-1}$, Air Liquide, 99.9999%, CAS: 7440-37-1) for 10 hours and the nitrate precursors were further decomposed at 623 K for 4 h under the $N_2$ flow (heating rate of 1 K $min^{-1}$). This impregnation, drying and decomposition procedure was repeated four times. For each impregnation step beyond the first one, the total mesopore volume was corrected by the volume of the metal species previously deposited in the pores, assuming a density of 6.1 $cm^3$ $g^{-1}$ ($Co_3O_4$). Finally, the catalyst was subjected to a thermal reduction treatment, after having placed it in the form of a packed bed inside a tubular reactor, in $H_2$ flow (200 $cm^3$ STP $min^{-1}$, Air Liquide, 99.9999%, CAS: 1333-74-0) at 673 K (2 K $min^{-1}$ to 423 K, followed by 0.83 K $min^{-1}$ to 673 K) for 5 h at atmospheric pressure, and then transferred and stored in a glove box, under exclusion of oxygen and water, until its further application in the inventive process. According to mercury intrusion porosimetry results, the fraction of the total pore volume which corresponds to macropores with diameters in the size range from 50 nm to 5 $\mu$m in the catalyst is 29% of the total volume of pores with diameters in the size range from 5 nm to 5 $\mu$m.

**Synthesis of a Fischer-Tropsch catalyst according to the invention (CoRu-NaPr/Al$_2$O$_3$_mM)**

An $Al_2O_3$ support material with a pore size distribution with similar contributions in the mesopore (peak at 10.7 nm in **Figure 2**) and macropore ranges (peak at 1989 nm in **Figure 2**), denoted hereafter as $Al_2O_3$_mM, was synthesized by calcination of a commercial microparticulate $\gamma$-$Al_2O_3$ (Versal, UOP) under a stagnant air atmosphere in a muffle oven at 823 K using a 0.5 K $min^{-1}$ heating rate from room temperature and sieving the resulting solid to retain particles in the size range of **< 0.08 mm** mm. The $Al_2O_3$ granules were first dried under dynamic vacuum (rotatory pump) at 423 K for 2 hours. Subsequently, the dry solid was impregnated under static vacuum with an aqueous solution containing $Co(NO_3)_2 \cdot 6H_2O$ (1.5 M, Sigma-Aldrich, ≥98%, CAS: 10026-22-9) and ruthenium (III) nitrosyl nitrate (in dilute nitric acid, Sigma-Aldrich, CAS: 34513-98-9) previously prepared to have an atomic ratio Ru/Co=0.007, and further acidified with 0.25 vol% $HNO_3$ (69-70 vol.% in $H_2O$, J.T. Baker, ≥ 99%, CAS: 7697-37-2). The volume of solution infiltrated in the $Al_2O_3$ support was equivalent to 90% of the total mesopore volume of the support as determined by $N_2$ physisorption. After impregnation, the solid was dried in the form of a packed bed in a tubular reactor at 343 K under vertical downward $N_2$ flow (200 $cm^3$ $g_{cat}^{-1}$ $min^{-1}$, Air Liquide, 99.9999%, CAS: 7440-37-1) for 10 hours and the nitrate precursors were further decomposed at 623 K for 4 h under the $N_2$ flow (heating rate of 1 K $min^{-1}$). This impregnation, drying and decomposition procedure was repeated once. Then, the aqueous solution containing $Co(NO_3)_2 \cdot 6H_2O$ (1.5 M) and ruthenium (III) nitrosyl nitrate (in dilute nitric acid), previously prepared to have an atomic ratio Ru/Co=0.007, and further acidified with 0.25 vol% $HNO_3$ (69-70 vol.% in $H_2O$) was diluted with 50wt% 0.5 M $HNO_3$. This solution was used for a third impregnation step, again filling 90% of the total mesopore volume of the support as determined by $N_2$ physisorption, followed by the drying and decomposition steps, as described above to produce a CoRu/Al$_2$O$_3$ material. For each impregnation step beyond the first one, the total mesopore volume was corrected by the volume of the metal species previously deposited in the pores, assuming a density of 6.1 $cm^3$ $g^{-1}$ ($Co_3O_4$).

Next, the as-calcined CoRu/Al$_2$O$_3$ material was dried as detailed above, and further impregnated by slurring it in an aqueous solution of the praseodymium (Pr) and sodium (Na) salts in order to introduce both Pr and Na as promoters. For each 1 g of CoRu/Al$_2$O$_3$ material to impregnate, 11.1 $cm^3$ of an aqueous solution containing 115.6 mg of $Pr(NO_3)_3 \cdot 6H_2O$ (Sigma-Aldrich, 99.9%, CAS: 15878-77-0) and 5.7 mg $NaNO_3$ $NaNO_3$ (Sigma-Aldrich, >99%, CAS: 7631-99-4) dissolved in 0.5 M $HNO_3$ were applied in this impregnation step. After slurring the solid in the impregnating solution under shaker stirring, water was removed in a rotary evaporator at 323 K and the resulting dry solid was transferred to a tubular reactor, packed in the form of a packed bed and calcined at 623 K for 4 h under vertical downward synthetic air flow (Air Liquide, 20.5 ± 0.5 mol% $O_2$ in $N_2$, 99.999%, CAS: 132259-10-0) using a heating rate of 1 K $min^{-1}$ from room temperature and a gas flow of 200 $cm^3$ $g_{solid}^{-1}$ $min^{-1}$. Finally, the catalyst was subjected to a thermal reduction treatment, after having placed it in the form of a packed bed inside a tubular reactor, in $H_2$ flow (200 $cm^3$ STP $min^{-1}$, Air Liquide, 99.9999%, CAS: 1333-74-0) at 673 K (2 K $min^{-1}$ to 423 K, followed by 0.83 K $min^{-1}$ to 673 K) for 5 h at atmospheric pressure, and then transferred and stored in a glove box, under exclusion of oxygen and water, until its further application in the inventive process. According to mercury intrusion porosimetry results, the fraction of the total pore volume which corresponds to macropores with diameters in the size range from 50 nm to 5 $\mu$m in the catalyst is 63% of the total volume of pores with diameters in the size range from 5 nm to 5 $\mu$m.

**General method I to integrate catalyst and perform a reaction test according to the inventive process**

In a general method of this invention, pre-set amounts of (i) the Fischer-Tropsch catalyst, (ii) a metal precursor for the hydroformylation catalyst, (iii) one or various organic compounds as ligand for the development of the hydroformylation catalysts, and (iv) a solvent, are added under an inert atmosphere, that is under the exclusion of oxygen and moisture, into a 10 mL borosilicate glass inlet in order: first (i), then (ii), (iii) and finally (iv). 50 mg of iso-octane (Sigma Aldrich, 98%) are added as internal standard for gas chromatography analysis. The glass-inlet is transferred inside a 12 mL stainless-steel autoclave discontinuous reactor equipped with a magnetic stirrer and the reactor is sealed under exclusion of air and moisture. Next, the reactor is flushed at least twice with 120 bar of a certified syngas mixture of $H_2$:CO:Ar with a molar composition 60:30:10 (Air Liquide) fed from a single pressurized gas cylinder. Finally, the reactor is pressurized to a total pressure of 120 bar, at room temperature, with the same syngas mixture. The reaction of conversion of syngas is then conducted by inserting the reactor in an aluminium heat distributor block on a heating-stirring plate preheated at 468 K and the stirring speed is set to 700 rpm. After a predefined reaction time, the reaction is quenched by letting the reactor cool down to room temperature inside a water bath.

**General method II to integrate catalyst and perform a reaction test according to the inventive process**

In another general method of this invention, pre-set amounts of (i) a solution of a metal precursor for the hydroformylation catalyst in the solvent, and (ii) a solution of one or various organic compounds as ligand for the development of the hydroformylation catalysts in the solvent, are added under an inert atmosphere, that is under the exclusion of oxygen and moisture, into a 10 mL borosilicate glass inlet in order: first (i), then (ii). 50 mg of iso-octane (Sigma Aldrich, 98%) are added as internal standard for gas chromatography analysis. The glass-inlet is transferred inside a 12 mL stainless-steel autoclave discontinuous reactor equipped with a magnetic stirrer and the reactor is sealed under exclusion of air and moisture. Next, the reactor is flushed at least twice with 120 bar of a certified syngas mixture of $H_2$:CO:Ar with a molar composition 60:30:10 (Air Liquide) fed from a single pressurized gas cylinder. Next, the reactor is pressurized to a total pressure of 120 bar with the same syngas mixture, and it is inserted in an aluminium heat distributor block on a heating-stirring plate preheated at 468 K and the stirring speed is set to 700 rpm. After 1 hour, the reactor is cooled to room temperature using a water bath and gas is released from the reactor to lower the total pressure to 2 bar. Then the reactor is transferred inside a glove box with an inert atmosphere, that is under exclusion of oxygen and moisture, and the remaining gas inside the reactor is released, the reactor is opened and a pre-set amount of the Fischer-Tropsch catalyst is added into the glass liner. Then the reactor is re-filled and pressurized with a certified syngas mixture of $H_2$:CO:Ar with a molar composition 60:30:10 up to a total pressure of 120 bar at room temperature. The reaction of conversion of syngas is then conducted by inserting the reactor in an aluminium heat distributor block on a heating-stirring plate preheated at 468 K and the stirring speed is set to 700 rpm. After a predefined reaction time, the reaction is quenched by letting the reactor cool down to room temperature inside a water bath.

**General method to analyze the products of a reaction of syngas conversion according to the inventive process**

After terminating the reaction of conversion of syngas, a portion of the gas phase inside the reactor was released to flush and fill two sampling loops, connected in series, of an Agilent 7890B gas chromatograph (GC). One of the sampling loops is injected into a capillary column (Restek RTX-1, 60 m) which elutes into an Flame Ionization detector (FID). The other sampling loop is injected into two consecutive packed-bed columns (HS-Q 80/120, 1x m + 1x 3 m) in series, which elute into a Thermal Conductivity detector (TCD) for the analysis of $H_2$, $CO_2$, and $C_2$-$C_3$ hydrocarbons. Along the same analysis channel, a molecular sieve column (13X) is used for the separation of Ar, $CH_4$, and CO permanent gases, which are detected using an additional TCD. After sample injection, the GC oven temperature is kept at 308 K for 10 min, after that the oven is heated to 483 K (with a heating ramp of 283 K min$^{-1}$), after which the temperature was kept at 483 K for 45 min. CO, $CH_4$, and $CO_2$ were quantified using TCD response factors relative to Ar, whereas $C_{2+}$ hydrocarbons and alcohols were quantified in the FID relative to $CH_4$.

Condensed phases in the reactor after the reaction test are collected and then centrifuged in an ultracentrifuge at 14.000 rpm, and the liquid and solid phases separated and recovered separately. The resulting liquid sample is injected into an Agilent 7890B gas chromatograph equipped with a capillary column (Restek RTX-1, 60 m) which elutes into an FID. Following injection, the GC oven temperature was increased from room temperature to 493 K at a heating rate of 278 K min$^{-1}$, after which temperature was kept at 493 K for 50 min. Hydrocarbons and alcohols were quantified based on their FID signal relative to iso-octane as standard.

The amount of wax hydrocarbons contained in the solid pellet recovered after centrifugation are quantified by thermogravimetric analysis (TGA) in air using a corundum crucible inside a Netzsch Jupiter STA 449 C thermobalance and applying a heating ramp of 283 K min$^{-1}$ from 298 K to 1023 K. The fraction of the total amount of combusted matter associated to waxy hydrocarbon reaction products was determined by subtracting contributions from (i) the release of CO ligands in $Co(CO)_8$ on the basis of the cobalt carbonyl stoichiometry, the Co/Al stoichiometry in the Fischer-Tropsch catalyst and the total cobalt content in the inorganic residue left behind in the crucible after the TGA experiment as determined by energy-dispersive X-ray analysis (EDX) in a Hitachi S-3500N scanning electron microscope equipped with an Oxford Instruments EDS; and (ii) the combustion of organic ligands from the hydroformylation catalyst as determined from the total phosphorous content in the ligand and the ligand-to-cobalt stoichiometry in the molecular hydroformylation catalyst.

**Examples**

Example 1

In one example according to the inventive process, 30.5 mg of CoRu/Al$_2$O$_3$_m was applied as Fischer-Tropsch catalyst. The hydroformylation catalyst was developed from 9.0 mg Co$_2$(CO)$_8$ as metal precursor and 14.7 mg P(Cy)$_3$ as organic ligand. 1.37 mL of iso-hexane (2-methylpentane) was applied as solvent. The reaction integration of the catalysts and the syngas conversion reaction test were performed according to the general method II.

Example 2

In another example according to the inventive process, 41.5 mg of CoRu-NaPr/Al$_2$O$_3$_MM was applied as Fischer-Tropsch catalyst. The hydroformylation catalyst was developed from 24.0 mg Co$_2$(CO)$_3$ as metal precursor and 39.2 mg P(Cy)$_3$ as organic ligand. 1.37 mL of iso-hexane (2-methylpentane) was applied as solvent. The reaction integration of the catalysts and the syngas conversion reaction test were performed according to the general method II.

Example 3

In another example according to the inventive process, 39.7 mg of CoRu-NaPr/Al$_2$O$_3$_mM was applied as Fischer-Tropsch catalyst. The hydroformylation catalyst was developed from 12.0 mg Co$_2$(CO)$_8$ as metal precursor and 19.6 mg P(Cy)$_3$ as organic ligand. 1.37 mL of iso-hexane (2-methylpentane) was applied as solvent. The reaction integration of the catalysts and the syngas conversion reaction test were performed according to the general method II.

Example 4

In another example according to the inventive process, 43.4 mg of CoRu-NaPr/Al$_2$O$_3$_mM was applied as Fischer-Tropsch catalyst. The hydroformylation catalyst was developed from 12.0 mg Co$_2$(CO)$_8$ as metal precursor and 19.6 mg P(Cy)$_3$ as organic ligand. 1.37 mL of iso-hexane (2-methylpentane) was applied as solvent. The reaction integration of the catalysts and the syngas conversion reaction test were performed according to the general method II.

Example 5

In another example according to the inventive process, 40.0 mg of CoRu-NaPr/Al$_2$O$_3$_mM was applied as Fischer-Tropsch catalyst. The hydroformylation catalyst was developed from 12.0 mg Co$_2$(CO)$_8$ as metal precursor and 18.5 mg P(Ph)$_3$ as organic ligand. 1.37 mL of iso-hexane (2-methylpentane) was applied as solvent. The reaction integration of the catalysts and the syngas conversion reaction test were performed according to the general method II. The abbreviation P(Ph)$_3$ stands for tri-phenyl phosphine.

Example 6

In another example according to the inventive process, 41.3 mg of CoRu-NaPr/Al$_2$O$_3$_mM was applied as Fischer-Tropsch catalyst. The hydroformylation catalyst was developed from 23.9 mg Co$_2$(CO)$_8$ as metal precursor and 39.2 mg P(Cy)$_3$ as organic ligand. 1.0 mL of iso-pentane (2-methylbutane) was applied as solvent. The reaction integration of the catalysts and the syngas conversion reaction test were performed according to the general method I.

Example 7

In another example according to the inventive process, 41.8 mg of CoRu-NaPr/Al$_2$O$_3$_mM was applied as Fischer-Tropsch catalyst. The hydroformylation catalyst was developed from 12.0 mg Co$_2$(CO)$_8$ as metal precursor and 15.0 mg P(n-Bu)$_3$ as organic ligand. 1.37 mL of iso-hexane (2-methylpentane) was applied as solvent. The reaction integration of the catalysts and the syngas conversion reaction test were performed according to the general method II. The abbreviation P(n-Bu)$_3$ stands for tri-n-butyl phosphine.

Example 8

In another example according to the inventive process, 40.2 mg of CoRu-NaPr/Al$_2$O$_3$_mM was applied as Fischer-Tropsch catalyst. The hydroformylation catalyst was developed from 12.0 mg Co$_2$(CO)$_8$ as metal precursor and 19.6 mg P(Cy)$_3$ as organic ligand. 1.37 mL of iso-hexane (2-methylpentane) was applied as solvent. The reaction integration of the catalysts and the syngas conversion reaction test were performed according to the general method II.

Comparative Example CP 1

In a comparative example not according to the inventive process, 40.7 mg of CoRu-NaPr/Al$_2$O$_3$_mM was applied as Fischer-Tropsch catalyst. As a hydroformylation catalyst only 12.0 mg Co$_2$(CO)$_8$ were added to the reaction pot, without the addition of any organic ligand component. 1.37 mL of iso-hexane (2-methylpentane) was applied as solvent. Otherwise, the reaction integration of the catalysts and the syngas conversion reaction test were performed according to the general method II.

Comparative Example CP 2

In a comparative example not according to the inventive process, 40.0 mg of CoRu-NaPr/Al$_2$O$_3$_mM was applied as Fischer-Tropsch catalyst. No hydroformylation catalyst was added to the reaction pot. 1.37 mL of iso-hexane (2-methylpentane) was applied as solvent. Otherwise, the syngas conversion reaction test were performed according to the general method I.

Comparative Example CP 3

In a comparative example not according to the inventive process, no Fischer-Tropsch catalyst was added to the reaction pot. The hydroformylation catalyst was developed from 12.0 mg Co$_2$(CO)$_8$ as metal precursor and 14.1 mg P(Cy)$_3$ as organic ligand. 1.37 mL of iso-hexane (2-methylpentane) was applied as solvent. Otherwise, the syngas conversion reaction test were performed according to the general method II.

**Table 1**

| Example | T[a] (K) | Time[b] (h) | X$_{CO}$[c] (%) | Selectivity CO$_2$ (%) | Selectivity C$_{3+}$-ROH[d] (%) | Time-yield C$_{3+}$-ROH[e] (mg/g$_{Co}$·h$^{-1}$) | α$_{ROH}$[f] (-) |
|---|---|---|---|---|---|---|---|
| 1 | 473 | 24 | 24.5 | 1.1 | 32.2 | 93.6 | 0.66[g] |
| 2 | 473 | 24 | 31.0 | 1.5 | 44.3 | 80.0 | 0.65 |
| 3 | 473 | 24 | 27.6 | 1.9 | 47.5 | 77.0 | 0.67 |
| 4 | 473 | 32 | 45.8 | 1.1 | 47.8 | 132.5 | 0.75[g] |
| 5 | 473 | 24 | 28.8 | 2.3 | 44.8 | 104.3 | 0.65 |
| 6 | 473 | 24 | 32.3 | 1.5 | 47.0 | 99.4 | 0.77 |
| 7 | 473 | 24 | 26.8 | 4.9 | 51.3 | 113.3 | 0.69 |
| 8 | 473 | 48 | 47.9 | 2.7 | 48.1 | 82.3 | 0.68 |
| CP 1 | 473 | 24 | 41.8 | 1.6 | 18.1 | 62.8 | 0.60 |
| CP 2 | 473 | 24 | 36.1 | 1.5 | 24.2 | 79.0 | 0.61 |
| CP 3 | 473 | 24 | 0.2 | ≥60 | 0.0 | 0.0 | - |

[a] Temperature; [b] Reaction time; [c] Conversion of CO; [d] Selectivity to higher alcohols with three or more carbon atoms (C$_{3+}$-ROH); [e] Cobalt mass-specific time-yield to higher alcohols with three or more carbon atoms; [f] Anderson-Schulz-Flory chain-growth probability for alcohols as determined for alcohol products in the chain length range of 3-8 carbon atoms, unless otherwise stated. [g] Determined for alcohol products in the chain length range of 4-9 carbon atoms.

As it can be seen from the results presented in Table 1, the inventive examples illustrate a process whereby syngas is converted to alcohols with high selectivity and time-yield. Under otherwise identical process conditions, the application of a solvent which is under supercritical conditions at the applied process conditions, such as iso-pentane (2-methylbutane, with critical temperature T$_c$=461 K, critical pressure P$_c$=33 bar) in Example 6, leads to enhanced selectivity to higher alcohols compared to the use of a solvent, iso-hexane (2-methyl-pentane, with critical temperature T$_c$=498 K, critical pressure P$_c$=30 bar), which is under sub-critical conditions at the applied process conditions, in Example 2. Comparative examples, not according to the present invention, lead to significantly lower selectivity and/or time-yield to C$_{3+}$ alcohols.

**Claims**

1.  Process for converting a syngas feed stream into $C_{3+}$ alcohols wherein,
    in a first step, a syngas feed stream comprising carbon monoxide and hydrogen with a $H_2/CO$ ratio in the range of 0.4 to 4.0 is contacted, in a solvent in a single reactor in a temperature range of 298 K to 533 K under a reaction pressure of 1 bar to 300 bar with catalyst combination comprising

    (i) a Fischer-Tropsch catalyst as a first catalyst, which converts syngas to a hydrocarbon mixture comprising $C_{2+}$ olefins and which is essentially inactive for the water-gas-shit reaction and,
    (ii) a hydroformylation catalyst as a second catalyst, which is stable and active for the reductive hydroformylation of olefins and which is highly selective for the production of terminal alcohols;
    in a ratio between the Fischer-Tropsch catalyst and the hydroformylation catalyst, expressed as the molar ratio of the metal in the Fischer-Tropsch catalyst and the metal in the hydroformylation is in the range of 30:1-1:4, and,

    in a second step, $C_{3+}$ alcohols formed as reaction products are recovered from the reactor.

2.  Process for converting a syngas feed stream into $C_{3+}$ alcohols according to claim 1, wherein a Fischer-Tropsch catalyst is used which has an activity for the Fischer-Tropsch synthesis expressed as a metal mass-specific rate of CO conversion, being equal to or higher than 5 mmolCO $g_{metal}^{-1}$ $h^{-1}$, wherein the Fischer-Tropsch catalyst delivers a selectivity to $CO_2$ equal to or lower than 5% on a carbon basis, and a molar abundance of alpha-olefin hydrocarbons in the hydrocarbon products with hydrocarbon chain lengths in the range of $C_3$-$C_{10}$ which is equal to or greater than 30% on a carbon basis, when a syngas feed with a $H_2/CO$ molar ratio of 2.0 is contacted with the Fischer-Tropsch catalyst at a reaction temperature equal to or lower than 483 K, and a reaction pressure equal to or greater than 15 bar and the CO conversion achieved in the reactor is greater than 15%.

3.  Process for converting a syngas feed stream into $C_{3+}$ alcohols according to any one of claims 1 or 2, wherein the Fischer-Tropsch catalyst is a supported metal catalyst, wherein a metal selected from Co, Ru or a combination thereof is supported on a porous carrier selected from oxide carriers selected from $Al_2O_3$, $SiO_2$, $TiO_2$, or any combination thereof, carbide or oxy-carbide materials selected from SiC, $SiO_xC_y$, with x being in the range of 0<x<2 and y in the range of 0<y<1, pure carbon or any combination thereof.

4.  Process for converting a syngas feed stream into $C_{3+}$ alcohols according to claim 3, wherein the porous carrier comprises mesopores and macropores wherein the volume of macropores preferably accounts for at least a 5 Vol.-% of the total pore volume, more preferably for at least a 20 Vol.-% of the total pore volume.

5.  Process for converting a syngas feed stream into $C_{3+}$ alcohols according to any one of the preceding claims wherein the Fischer-Tropsch catalyst is additionally comprising a metal promoter selected from ruthenium, rhenium, platinum, palladium, silver, gold, copper, wherein the weight content of the promoter is preferably larger than 0 wt% and lower than 10 wt%, more preferably larger than 0 wt% and lower than 2 wt%, calculated on the basis of the total mass of the Fischer-Tropsch catalyst.

6.  Process for converting a syngas feed stream into $C_{3+}$ alcohols according to claim 5 wherein the Fischer-Tropsch catalyst is additionally comprising a second promoter which can be can be selected from alkali metals, alkaline earth metals, boron, aluminium, gallium, indium, carbon, silicon, germanium, tin, nitrogen, phosphorous, arsenic, antimony, lanthanides, transition metals, or any combination thereof whereby said second promoter is present in elementary form or in ionic form as a salt and is used in a weight content of the second promoter preferably larger than 0 wt% and lower than 50 wt%, more preferably larger than 0 wt% and lower than 20 wt%, calculated on the basis of the total mass of the Fischer-Tropsch catalyst.

7.  Process for converting a syngas feed stream into $C_{3+}$ alcohols according to any one of the preceding claims wherein a hydroformylation catalyst is used as a second catalyst which comprises (i) a metal selected from cobalt, ruthenium, rhodium, iridium, or any combination thereof and (ii) at least one organic ligand which binds to the metal to form a coordination complex in a molar ratio ligand/metal of 1 to 5.

8.  Process for converting a syngas feed stream into $C_{3+}$ alcohols according to any one of the preceding claims wherein a hydroformylation catalyst is used which is prepared by reacting, in an organic solvent, at least one metal precursor compound selected from carbonyl complexes or salts, preferably selected from formate, acetate, acetylacetonate, carboxylate, carbonate, oxalate, halide, amine, imide, and any combination thereof, of cobalt, ruthenium, rhodium, iridium or any combination thereof with at least one organic ligand which binds to the metal to form a coordination complex and is preferably selected from the group of oxygen-containing ligands, phosphorus-containing ligands, nitrogen-containing ligands, arsenic-containing ligands and combinations thereof, and preferably is a phosphorus-containing ligand.

9. Process for converting a syngas feed stream into $C_{3+}$ alcohols according to any one of claims 1 to 8, wherein the reactor is operated in a batch mode.

10. Process for converting a syngas feed stream into $C_{3+}$ alcohols according to any one of claims 1 to 8, wherein the reactor is operated in a continuous mode, wherein at least one stream of the gas feed and one stream of the solvent are provided continuously to the reactor inlet and a product stream is continuously removed at the outlet of the reactor.

11. A catalyst combination for converting a syngas feed into $C_{3+}$ alcohols comprising

(i) a Fischer-Tropsch catalyst as a first catalyst, which converts syngas to a hydrocarbon mixture comprising $C_{2+}$ olefins and which is essentially inactive for the water-gas-shit reaction and,
(ii) a hydroformylation catalyst as a second catalyst, which is stable and active for the reductive hydroformylation of olefins and which is highly selective for the production of terminal alcohols;

in a ratio between the Fischer-Tropsch catalyst and the hydroformylation catalyst, expressed as the molar ratio of the metal in the Fischer-Tropsch catalyst and the metal in the hydroformylation is selected in the range of 30:1-1:4, more preferably in the range of 10:1-1:2, and most preferably in the range of 4:1-1:1.

12. Use of a catalyst combination as claimed in claim 11 in a single-pot process for converting a syngas feed stream into $C_{3+}$ alcohols.

Figure 1

Figure 2

Europäisches
Patentamt
European
Patent Office
Office européen
des brevets

# EUROPEAN SEARCH REPORT

Application Number

EP 21 18 3582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHANG JIANLI ET AL: "Insight into the role of Co2C supported on reduced graphene oxide in Fischer-Tropsch synthesis and ethene hydroformylation", APPLIED CATALYSIS A: GENERAL, ELSEVIER, AMSTERDAM, NL, vol. 614, 18 February 2021 (2021-02-18), XP086515092, ISSN: 0926-860X, DOI: 10.1016/J.APCATA.2021.118050 [retrieved on 2021-02-18] * 2. Materials and methods; figure 6; table 2 * ----- | 1-4,9-12 | INV. B01J35/00 B01J23/89 B01J31/24 B01J31/20 C07C29/156 C07C45/50 |
| X | MAX SCHALLER ET AL: "Iron-Based Fischer-Tropsch Catalysts for Higher Alcohol Synthesis", CHEMIE INGENIEUR TECHNIK, WILEY VCH. VERLAG, WEINHEIM; DE, vol. 90, no. 5, 1 March 2018 (2018-03-01), pages 713-720, XP071103667, ISSN: 0009-286X, DOI: 10.1002/CITE.201700154 * figure 1; table 1 * ----- | 11,12 | |
| A | US 6 756 411 B2 (SASOL TECH PTY LTD [ZA]) 29 June 2004 (2004-06-29) * claims * ----- | 1-12 | TECHNICAL FIELDS SEARCHED (IPC) B01J C07C |

The present search report has been drawn up for all claims

| Place of search | Date of completion of the search | Examiner |
|---|---|---|
| Munich | 15 December 2021 | Mauger, Jeremy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT
## ON EUROPEAN PATENT APPLICATION NO.

EP 21 18 3582

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2021

| Patent document cited in search report | | Publication date | Patent family member(s) | | Publication date |
|---|---|---|---|---|---|
| US 6756411 | B2 | 29-06-2004 | US | 2003120118 A1 | 26-06-2003 |
| | | | US | 2004167235 A1 | 26-08-2004 |
| | | | US | 2005282916 A1 | 22-12-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1658254 A2
- US 7674940 B2
- WO 2002100806 A1
- CN 107744810 A
- US 4513100 A
- WO 2012062338 A1
- US 4598061 A
- US 4096164 A
- CN 1179993 A
- WO 2006123150 A1
- WO 2009032982 A2
- CN 101535217 B
- WO 2012078277 A1
- US 4122110 A
- US 4291126 A

**Non-patent literature cited in the description**

- Hydroformylation: Fundamentals, Processes, and Applications in Organic Synthesis. 2016
- **H.T. LUK et al.** Status and prospects in higher alcohols synthesis from syngas. *Chem. Soc. Rev.,* 2017, vol. 46, 1358-1426
- **COURTY et al.** C1-C6 alcohols from synthesis gas on copper-cobalt catalysts. *J. Mol. Catal.,* 1982, vol. 17, 241-254
- **XIANG et al.** Long-Chain Terminal Alcohols through Catalytic CO Hydrogenation. *J. Am. Chem. Soc.,* 2013, vol. 135, 7114-7117
- **XIANG et al.** Higher alcohols through CO hydrogenation over CoCu catalysts: influence of precursor activation. *ACS Catal.,* 2014, vol. 4 (8), 2792-2800
- **PRIETO et al.** Design and synthesis of copper-cobalt catalysts for the selective conversion of synthesis gas to ethanol and higher alcohols. *Angew. Chem. Int. Ed.,* 2014, vol. 53, 6397-6401
- **ZHAO et al.** Insight into the Formation of Co@Co2C Catalysts for Direct Synthesis of Higher Alcohols and Olefins from Syngas. *ACS Catal.,* 2018, vol. 8, 228-241
- Handbook of Heterogeneous Catalysis. Wiley-VCH Verlag GmbH & Co. Weinheim, 2008, vol. 1
- Synthesis of solid catalysts. Wiley-VCH Verlag GmbH & Co. Weinheim, 2009
- *CHEMICAL ABSTRACTS,* 10026-22-9
- *CHEMICAL ABSTRACTS,* 34513-98-9
- *CHEMICAL ABSTRACTS,* 7697-37-2
- *CHEMICAL ABSTRACTS,* 7440-37-1
- *CHEMICAL ABSTRACTS,* 1333-74-0
- *CHEMICAL ABSTRACTS,* 15878-77-0
- *CHEMICAL ABSTRACTS,* 7631-99-4
- *CHEMICAL ABSTRACTS,* 132259-10-0